# FASCICULE DE BREVET EUROPEEN

(11) **EP 4 259 092 B1**
(45) Date de publication et mention de la délivrance du brevet: **18.02.2026**
(21) Numéro de dépôt: 21816481.2
(22) Date de dépôt: 30.11.2021
(51) Int. Cl.: A61K 9/00, C08J 3/12, C08J 3/20, C08J 3/24, C08G 69/40, C08G 69/48, C08G 69/10

(54) **PROCÉDÉ DE PRÉPARATION D'UNE COMPOSITION PHARMACEUTIQUE À USAGE TOPIQUE COMPRENANT COMME AGENT ÉPAISSISSANT UN POLYMÈRE SE PRÉSENTANT SOUS LA FORME D'UN SOLIDE PULVÉRULENT**
VERFAHREN ZUR HERSTELLUNG EINER PHARMAZEUTISCHEN ZUSAMMENSETZUNG ZUR TOPISCHEN ANWENDUNG, DIE EIN POLYMER IN FORM EINES PULVERFÖRMIGEN FESTSTOFFES ALS VERDICKUNGSMITTEL ENTHÄLT
PROCESS FOR THE PREPARATION OF A PHARMACEUTICAL COMPOSITION FOR TOPICAL USE COMPRISING A POLYMER IN THE FORM OF A PULVERULENT SOLID AS THICKENING AGENT

(30) Priorité: 14.12.2020 FR 2013175
(43) Date de publication de la demande: 18.10.2023
(73) Titulaire: Société d'Exploitation de Produits pour les Industries Chimiques SEPPIC, 75321 Paris cedex 07 (FR)
(72) Inventeur: BODOC, Miruna, 81100 CASTRES (FR); MONTEILLET, Stéphane, 81100 CASTRES (FR)
(74) Mandataire: Air Liquide
(86) Numéro de dépôt international: PCT/EP2021/083622
(87) Numéro de publication internationale: WO 2022/128453

(56) Documents cités:
- WO-A1-2018/222545
- XIAOLI YANG ED - QUAN PHD DR XIE: "Preparation and Characterization of -Poly(Glutamic Acid) Copolymer with Glycol Diglycidyl Ether", PROCEDIA ENVIRONMENTAL SCIENCES, vol. 8, 25 November 2011 (2011-11-25) - 26 November 2011 (2011-11-26), pages 11 - 15, XP028338556, ISSN: 1878-0296, [retrieved on 20111207], DOI: 10.1016/J.PROENV.2011.10.004
- LI ZHENG ET AL: "Preparation of [gamma]-PGA hydrogels and swelling behaviors in salt solutions with different ionic valence numbers", RSC ADVANCES, ROYAL SOCIETY OF CHEMISTRY, GB, vol. 7, no. 18, 13 February 2017 (2017-02-13), pages 11085 - 11093, XP009528837, ISSN: 2046-2069, DOI: 10.1039/C6RA26419K

## Description

La présente invention est relative à un procédé de préparation d'une composition pharmaceutique (F) à usage topique comprenant comme agent épaississant un polymère (P) se présentant sous la forme d'un solide pulvérulent.

Les polymères sont largement utilisés aujourd'hui dans les compositions pharmaceutiques à usage topique et représentent la deuxième famille de produits les plus utilisés dans ce type de compositions. Les compositions pharmaceutiques à usage topique contiennent des phases polaires comme par exemple des phases constituées d'eau, et nécessitent dans la plupart des cas l'utilisation d'agents modificateurs de rhéologie, comme par exemple des polymères, pour augmenter la viscosité de ces phases polaires, ainsi que pour conférer un comportement rhéologique bien défini.

Parmi les polymères modificateurs de rhéologie des phases polaires, nous pouvons citer des polymères naturels, comme par exemple des polysaccharides à bases d'oses ou des polysaccharides à base de dérivés d'oses, ou bien des polymères synthétiques, de type polyélectrolytes, anioniques ou cationiques, amphiphiles, linéaires ou ramifiés, réticulés ou non réticulés. Majoritairement présents sur le marché, les polymères synthétiques, présentent la propriété de se déployer dans la phase polaire, sous l'effet des répulsions électrostatiques dues à la présence des charges (négatives et/ou positives) sur le squelette polymérique, linéaire ou ramifié, non-réticulé ou réticulé. Ces modificateurs de rhéologie apportent à la fois une augmentation de la viscosité de la phase polaire, ainsi qu'une certaine consistance et/ou un effet stabilisant conféré à la composition pharmaceutique à usage topique.

Afin de répondre aux besoins des formulateurs et d'améliorer les performances, les différents travaux scientifiques récents ont relaté la mise au point de nouveaux systèmes polymériques innovants et variés. Ainsi, les polymères utilisés à la destination des industries de la détergence, peuvent jouer un rôle fonctionnel en tant qu'agents filmogènes, agents modificateurs de rhéologie, permettant la stabilisation des phases grasses dans les émulsions de type eau-dans-huile et de type huile-dans-eau, la stabilisation de particules solides (pigments et charges) ou en tant qu'agents ayant un impact sur l'aspect de la formule (transparence, translucide, opacité). Les polymères modificateurs de rhéologie de phase polaires, et plus particulièrement de phases aqueuses, sont principalement des polyélectrolytes, qui résultent de la polymérisation radicalaire de monomères de type (méth)acrylique , c'est-à-dire l'acide acrylique ou l'acide méthacrylique, d'esters dérivés de l'acide acrylique ou de l'acide méthacrylique, ou encore des dérivés d'acrylamide ou de méthacrylamide.

Développer de nouveaux modificateurs de rhéologie biosourcés et biodégradables, aussi performants que les polymères synthétiques actuellement utilisés, constitue encore à ce jour un défi majeur. En effet, jusqu'à présent les solutions majoritairement utilisées pour épaissir des phases aqueuses mettent en jeu des ingrédients provenant de matières pétrochimiques et notamment de l'acide acrylique et de ses dérivés, de l'acide méthacrylique et de ses dérivés. Compte-tenu de la préoccupation croissante des consommateurs pour une économie et un développement durable et responsable, la substitution des matières premières d'origine pétrochimique par des matières premières d'origine renouvelable pour préparer des polymères, est un axe de recherche prioritaire.

A ce jour, il est décrit dans la littérature l'utilisation de différents polymères naturels ou provenant de matières premières renouvelables, dont les unités monomériques sont issues de la famille des sucres (glucose, arabinose, xylose, galactose, mannose, ribose, acide glucuronique, etc...) ou de la famille des acides aminés (acide glutamique, acide aspartique, lysine, etc...). Ces polymères sont majoritairement linéaires ou ramifiés selon le végétal dont ils sont issus ou selon leur procédé de fabrication.

A titre d'exemple de polymère d'origine naturelle, on peut citer l'acide polyglutamique (PGA) qui fait aujourd'hui l'objet de nombreux travaux de recherches. Il s'agit d'un polymère majoritairement linéaire et constitué d'unités monomériques acide glutamique (GA). L'acide glutamique est un acide aminé se caractérisant par une fonction amine en position α et par deux fonctions acides carboxyliques (ou carboxylates en fonction du pH) en positions α et γ (cf formule chimique n°1).

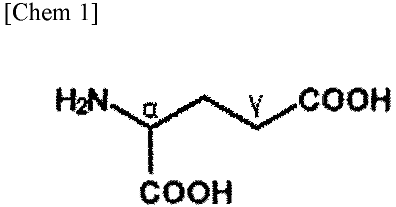

Structure chimique de l'acide glutamique (GA).

Une des voies pour augmenter la ramification d'un polymère synthétique, naturel ou d'origine naturelle consiste à réaliser des réactions de réticulation. La réticulation des chaînes polymériques a pour but de relier plusieurs chaînes polymériques les unes aux autres qui, lorsqu'ajoutées à une phase polaire, et plus particulièrement à de l'eau, se présentent à l'état d'un réseau tridimensionnel insoluble dans l'eau, mais gonflable à l'eau et conduisant alors à l'obtention d'un gel chimique.

La préparation de polymères réticulés peut s'effectuer :
- En une étape par la mise en réaction des monomères et de l'agent de réticulation pendant la réaction de polymérisation, ou bien
- En au moins deux étapes, dont la première consiste à préparer le polymère, et la deuxième consiste à faire réagir le polymère avec un agent de réticulation pour obtenir un polymère réticulé.

Il existe différentes réactions de réticulation de l'acide polyglutamique (PGA), ce qui permet d'obtenir des polymères d'origine naturelles avec des propriétés épaississantes en milieux polaires, notamment aqueux, améliorées. Parmi les agents réticulants connus pour être utilisés dans la réaction de réticulation du (PGA), les dérivés polyépoxydes sont les plus décrits, car ils permettent de mettre en oeuvre des procédés de réticulation dans des conditions respectueuses de l'environnement (température modérées, réaction en milieux aqueux et en l'absence de solvants nocifs).

Cependant, la mise en oeuvre de ces procédés nécessite de diluer le (PGA) à des taux élevés, ce qui conduit à l'obtention d'un gel aqueux comprenant pour 100% de sa masse une teneur massique inférieure ou égale à 10% d'un polymère (P) et difficile à mettre en œuvre par les formulateurs.

Partant de là, un problème qui se pose est de fournir une composition pharmaceutique à usage topique facile à utiliser comprenant des polymères d'origine naturelle et dont les matières premières sont renouvelables, et qui présentent des propriétés épaississantes de milieux polaires et particulièrement de milieux aqueux.

Une solution de la présente invention est un procédé de préparation d'une composition pharmaceutique (F) à usage topique comprenant au moins un principe actif pharmaceutique et comme agent épaississant et pour 100% de sa masse, entre 0,1% et 10% massique d'un polymère (P) se présentant sous la forme d'un solide pulvérulent constitué d'unités monomériques issues d'acide glutamique (GA) partiellement ou totalement salifié, et d'unités monomériques d'au moins un agent réticulant (AR) portant au moins deux fonctions glycidyle comprenant une étape (A) de préparation d'un polymère (P) et une étape (B) de mélange d'au moins un polymère (P) préparé lors de l'étape (A) avec au moins un principe actif pharmaceutique, comme défini à la revendication 1. L'étape c) de séchage par atomisation de la phase polaire (PP) issue de l'étape b) s'effectue au moyen d'un atomiseur mettant en œuvre un flux d'air et une buse dans lequel la température d'entrée du flux d'air est comprise entre 80°C et 180°C.

Par "solide pulvérulent", on désigne au sens de l'invention un solide constitué de fines particules peu ou pas liées entre elles.

Dans le polymère (P), les unités monomériques issues de l'acide glutamique (GA), partiellement ou totalement salifié, sont liées entre-elles :
- soit de telle sorte que la fonction amine d'une unité monomérique d'acide glutamique (GA) est liée de façon covalente avec la fonction carboxylique située en position alpha (α) d'une seconde unité monomérique d'acide glutamique (GA) ; le polymère résultant est alors nommé "acide α-polyglutamique" ou PAGA (cf formule chimique n° 2) partiellement ou totalement salifié,

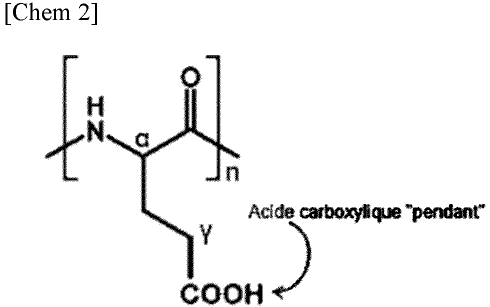

Structure chimique de l'acide α-polyglutamique ou PAGA.
soit de telle sorte que la fonction amine d'une unité monomérique d'acide glutamique (GA) est liée de façon covalente à la fonction carboxylique de la chaîne latérale située en position gamma (y) d'une seconde unité monomérique d'acide glutamique (GA); le polymère résultant est alors nommé "acide γ-polyglutamique" ou PGGA (cf formule chimique n° 3) partiellement ou totalement salifié.

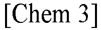

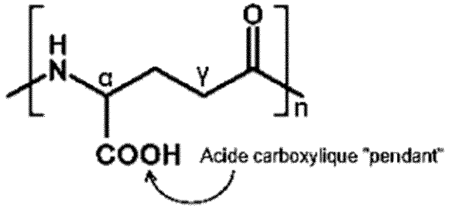

Structure chimique de l'acide γ-polyglutamique ou PGGA.

D'une manière générale, le PGA peut être préparé par voie chimique selon des procédés de synthèses peptidiques connus de l'homme du métier passant notamment par des étapes de protection(s) sélective(s), d'activation, de couplage et de déprotection(s). Le couplage consiste généralement en une attaque nucléophile de la fonction amine d'une unité monomérique d'acide glutamique sur une fonction acide carboxylique activée d'une autre unité monomérique d'acide glutamique.

Le PGGA peut également être obtenu selon des procédés comprenant au moins une étape de fermentation microbienne impliquant l'utilisation d'au moins une souche bactérienne.

Au sens de la présente invention, dans le polymère (P) tel que défini précédemment, le terme «salifié» indique que la fonction acide carboxylique "pendante" présente sur chaque unité monomérique d'acide glutamique (GA) du polymère (en position gamma dans le cas du PAGA ou alpha dans le cas du PGGA) se trouve sous une forme anionique ou carboxylate. Le contre-ion de cette fonction carboxylate est un cation dérivé, par exemples, de sels de métaux alcalins tels que le sodium, le potassium ou de sels de bases azotées tels que des amines, la lysine ou la monoéthanolamine (HO-CH₂- CH₂-NH₂).

Par "agent réticulant (AR) portant au moins deux fonctions glycidyles", on désigne au sens de la présente invention un agent réticulant (AR) tel que défini ci-dessus dont la structure moléculaire comprend au moins deux motifs ou fonctions glycidyles de formule (I') :

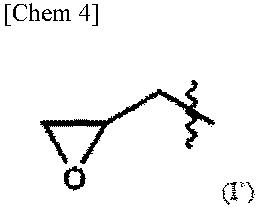

La réticulation des chaînes polymériques du polymère (P) s'effectue selon une réaction entre la fonction amine libre terminale (-NH2) et/ou une ou plusieurs fonctions carboxyliques ou carboxylates "pendantes" ou terminale (-COOH ou -COO⁻) présentes dans la structure dudit polymère (P), et au moins un groupe époxy présent dans la structure de l'agent réticulant (AR) portant au moins deux fonctions glycidyles.

Par « principe actif pharmaceutique », on désigne au sens de la présente invention une substance chimique qui entre dans la composition d'un médicament et qui démontre un effet thérapeutique ou prophylactique.

L'agent réticulant (AR) est choisi parmi les membres du groupe constitué par:
- Le Monoéthylène Glycol Diglycidyl Ether de formule (I) : 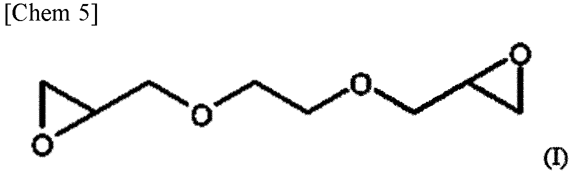
- Le composé de formule (II) :

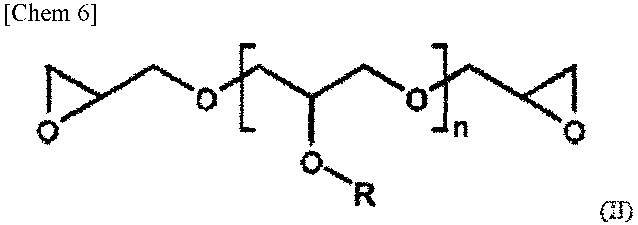

Avec R représentant un atome d'hydrogène ou le radical glycidyle 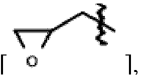 et n représentant un nombre entier supérieur ou égal à 1 et inférieur ou égal à 10 ;
- Le 1,3-propanediol Diglycidyl Ether de formule (III) : 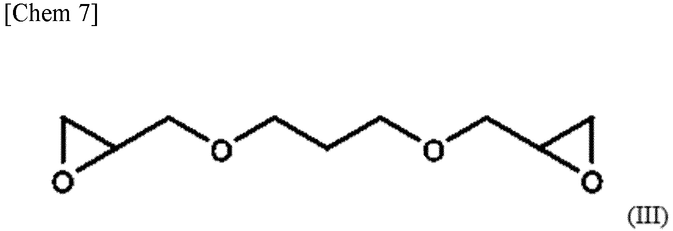
- Le 1,2-propanediol Diglycidyl Ether de formule (IV) : 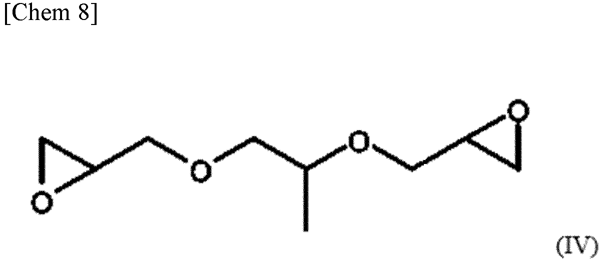
- Le 1,4-butanediol Diglycidyl Ether de formule (V) : 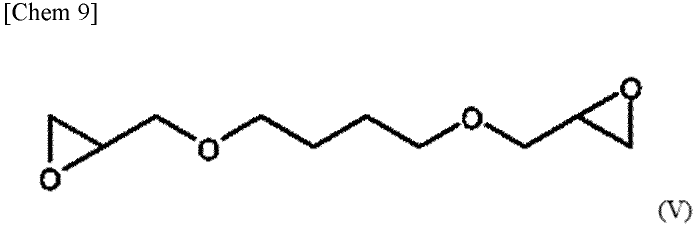
- le 1,2-butanediol Diglycidyl Ether de formule (VI) : 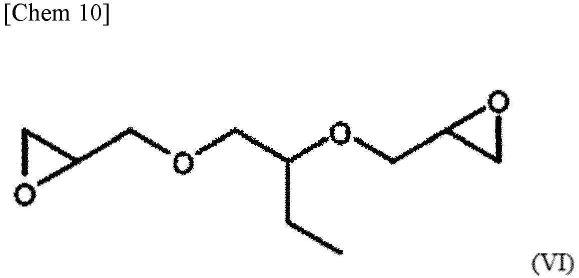
- Le 1,3-butanediol Diglycidyl Ether de formule (VII) : 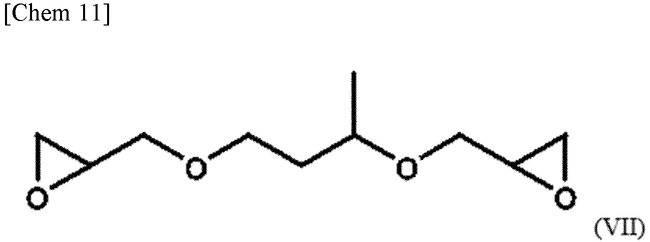
- Le 1,6-hexanediol Diglycidyl Ether de formule (VIII) : 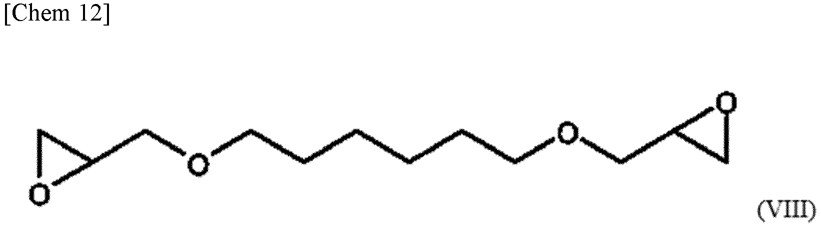
- Le composé de formule (IX) :

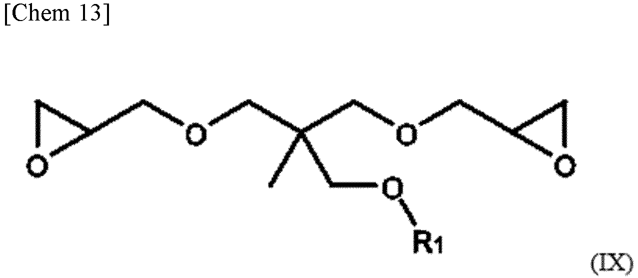

Avec R1 représentant un atome d'hydrogène ou le radical glycidyle 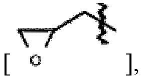
- Le composé de formule (X) :

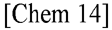

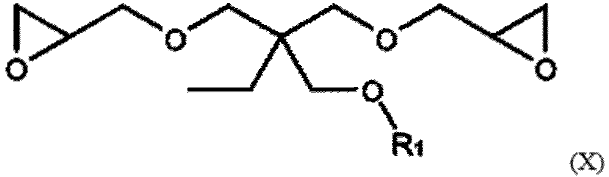

Avec R1 représentant un atome d'hydrogène ou le radical glycidyle 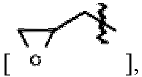
- Le composé de formule (XI) :

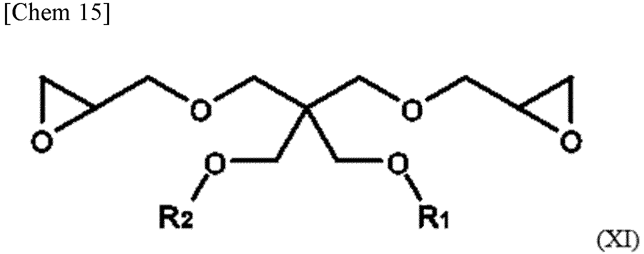

Avec R1 et R2, indépendants, représentant un atome d'hydrogène ou le radical glycidyle 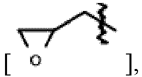
- Le composé de formule (XII) :

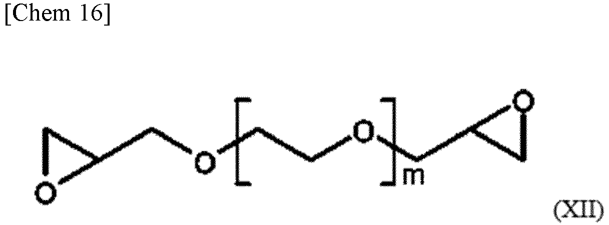

Avec m représentant un nombre entier supérieur ou égale à 2,
- Le composé de formule (XIII) :

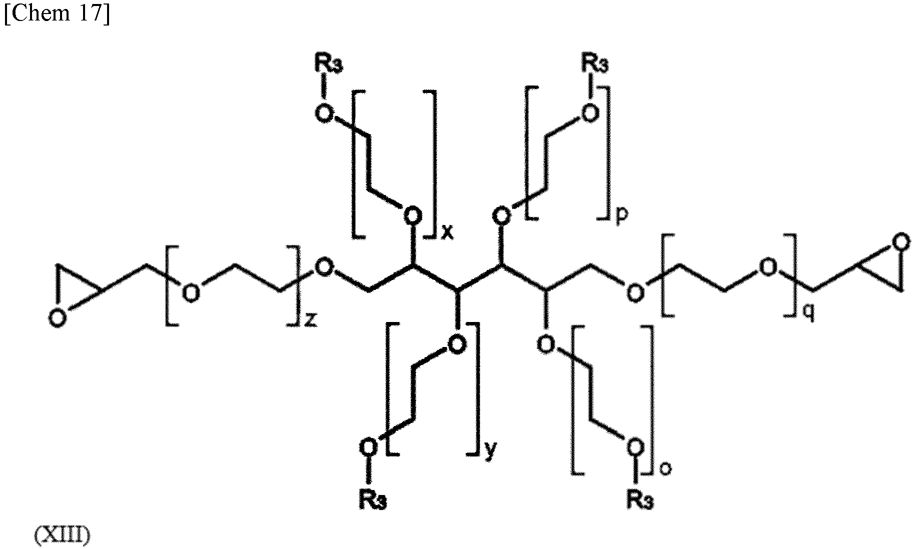

Avec R3 représentant un atome d'hydrogène ou le radical glycidyle 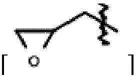 et x, y, z, o, p et q, indépendants les uns des autres, représentant un nombre entier supérieur ou égal à 2 et inférieur ou égal à 10.

Selon le cas, le procédé selon l'invention peut présenter également une ou plusieurs des caractéristiques suivantes :
- dans le polymère (P), pour 100% molaire d'unités monomériques issues de l'acide glutamique (GA), partiellement ou totalement salifié, l'agent réticulant (AR) représente de 0,5% à 20% molaire, plus particulièrement de 0,5% à 15% molaire, et encore plus particulièrement de 0,5% à 12% molaire .
- le polymère (P) comprend en outre un composé de formule (XIV) :

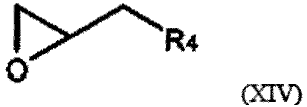

Avec R4 représentant un radical hydrocarboné linéaire ou ramifié, saturé ou insaturé, comportant optionnellement au moins une fonction hydroxyle fonctionnalisé ou non et comportant de 6 à 22 atomes de carbone.
- dans la formule (XIV), R4 représente un radical hydrocarboné choisi parmi les éléments du groupe constitué par le radical heptyle, octyle, nonyle, décyle, undécyle, undécényle, dodécyle, tridécyle, tétradécyle, pentadécyle, hexadécyle, heptadécyle, octadécyle, hydroxyoctadécyle, oléyle, linoléyle, linolényle, eicosyle et dodécosyle.
- dans la formule (XIV), R4 représente un radical hydrocarboné choisi parmi les éléments du groupe constitué par le radical n-octyle, n-nonyle, n-décyle, n-undécyle, n-undécényle, n-dodécyle, n-tétradécyle, n-hexadécyle, n-octadécyle, 12-hydroxyoctadécyle, n-eicosyle et n-dodécosyle.
- dans ledit polymère (P), pour 100% de la masse d'unités monomériques issues de l'acide glutamique (GA), partiellement ou totalement salifié, le composé de formule (XIV) représente de 1 % à 50% massique.
   Le PGGA peut exister sous différentes formes conformationnelles en solution dans l'eau. Celles-ci dépendent des liaisons hydrogènes inter et intra moléculaires et donc du pH, de la concentration en polymère, de la force ionique de la solution, ainsi que de la température. Les chaînes du PGGA peuvent ainsi adopter une forme d'hélice α, de feuillet β, d'agrégats ou alors se trouver dans un état désordonné et aléatoire. Selon un aspect particulier, le polymère (P) se trouve en conformation hélicoïdale lorsqu'il est présent dans une solution à une teneur massique inférieure ou égale à 0,1% et dont ladite solution aqueuse montre une valeur du pH inférieure ou égale à 7. Selon un aspect particulier, le polymère (P) se trouve en conformation feuillet lorsqu'il est présent dans une solution aqueuse à une teneur massique inférieure ou égale à 0,1% et dont ladite solution aqueuse montre une valeur du pH supérieure à 7.
- La composition pharmaceutique à usage topique (F) comprend pour 100% de sa masse entre 0,1% et 8% massique dudit polymère (P), plus particulièrement entre 0,1% et 5% massique.
- Le principe actif pharmaceutique est choisi parmi les agents antibactériens, les agents antimicrobiens, les agents antiparasitaires, les agents antihelminthiques, les agents anticoccidiens, les agents anti-cryptosporidiens, les agents anti-protozoaires, les agents antimycosiques, les agents anti-inflammatoires non stéroïdiens, les agents antiallergiques et immunomodulateurs, les agents analgésiques, les agents antihistaminiques, les agents anesthésiants locaux, les agents antisecticides, les agents antiseptiques, les agents antifongiques.

Selon le cas, le procédé selon l'invention peut présenter une ou plusieurs des caractéristiques suivantes :

### A l'étape a) :

- L'acide polyglutamique engagé est l'acide gamma polyglutamique (PGGA),
- L'ensemble des unités monomériques constituant le PGA est issu du glutamate de sodium, du glutamate de potassium, du glutamate d'ammonium, du glutamate de calcium, du glutamate de magnésium ou un mélange de ces formes,
- La phase polaire (PP) comprend pour 100% de sa masse : de 5% à 80% massique de l'acide polyglutamique (PGA) du polymère (P), de 0,025% à 8% massique de l'agent de réticulant (AR), de 12% à 94,975% massique d'au moins un solvant polaire,
- Le solvant polaire est choisi parmi les éléments du groupe constitué par l'eau, le méthanol, l'éthanol, le 1-propanol, le 2-propanol, l'isobutanol, le tertiobutanol, le 2-méthyl-2-propanol, le 1-butanol, le 2-butanol, l'acétone, le diméthyl cétone, le diéthyl cétone, le tétrahydropyrane, le tétrahydrofurane, le 2-méthyltétrahydrofurane, le 1,3-dioxane et le 1,4-dioxane.La phase polaire (PP) comprend en outre au moins un composé de formule (XIV) :

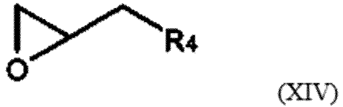

Avec R4 représentant un radical hydrocarboné linéaire ou ramifié, saturé ou insaturé, comportant optionnellement au moins une fonction hydroxyle et comportant de 6 à 22 atomes de carbone,

R4 représentant un radical hydrocarboné choisi parmi les éléments du groupe constitué par le radical heptyle, octyle, nonyle, décyle, undécyle, undécényle, dodécyle, tridécyle, tétradécyle, pentadécyle, hexadécyle, heptadécyle, octadécyle, hydroxyoctadécyle, oléyle, linoléyle, linolényle, eicosyle et dodécosyle.

Selon un aspect particulier, dans la formule (XIV), R4 représente un radical hydrocarboné choisi parmi les éléments du groupe constitué par le radical n-octyle, n-nonyle, n-décyle, n-undécyle, n-undécényle, n-dodécyle, n-tétradécyle, n-hexadécyle, n-octadécyle, 12-hydroxyoctadécyle, n-eicosyle et n-dodécosyle.
- La teneur en composé de formule (XIV) dans la phase polaire (PP) est comprise, pour 100% massique de ladite phase polaire (PP), entre 0,05% et 35% massique, étant entendu que la somme des proportions massiques de l'acide polyglutamique (PGA), de l'agent de réticulation (AR), du solvant polaire (SP) et du composé de formule (XIV) est égale à 100%.

### A l'étape b) :

- Le pH est compris entre 4 et 10, de préférence entre 5 et 7,

### A l'étape c) :

-
- La température d'entrée du flux d'air est comprise de préférence entre 100 et 170°C.

Selon un aspect particulier, la phase polaire (PP) comprend pour 100% de sa masse : de 5% à 70% massique de l'acide polyglutamique (PGA) du polymère (P), de 0,025% à 7% massique de l'agent de réticulant (AR), de 23% à 94,975% massique d'au moins un solvant polaire (SP). Selon un aspect encore plus particulier, la phase polaire (PP) comprend pour 100% de sa masse : de 5% à 60% massique de l'acide polyglutamique (PGA) du polymère (P), de 0,025% à 6% massique de l'agent de réticulant (AR), de 34% à 94,975% massique d'au moins un solvant polaire (SP).

Selon un autre aspect encore plus particulier, le solvant polaire (SP) est l'eau.

Selon un autre aspect particulier, R4 représentant un radical hydrocarboné est choisi parmi les éléments du groupe constitué par le radical n-octyle, n-nonyle, n-décyle, n-undécyle, n-undécényle, n-dodécyle, n-tétradécyle, n-hexadécyle, n-octadécyle, 12-hydroxy octadécyle, n-eicosyle et n-dodécosyle.

Selon un autre aspect particulier, la teneur en composé de formule (XIV) dans la phase polaire (PP) est comprise, pour 100% massique de ladite phase polaire (PP), entre 0,05% et 20% massique, et encore plus particulièrement entre 0,05% et 15% massique, étant entendu que la somme des proportions massiques de l'acide polyglutamique (PGA), de l'agent de réticulation (AR), du solvant polaire (SP) et du composé de formule (XIV) est égale à 100%.

### Exemples :

Les exemples suivants illustrent l'invention sans toutefois la limiter.

### Exemple 1 : Préparation d'un PGGA de sodium selon l'invention réticulé par du Polyéthylene glycol Diglycidyl Ether et atomisé :

Le procédé de synthèse comprend les 4 étapes suivantes :

### Etape 1) : Réalisation d'un gel aqueux de PGGA de sodium :

200 grammes d'eau déminéralisée sont placés sous agitation avec un agitateur mécanique de marque Rayneri^{™} et équipé d'un mobile de type défloculeuse et 20 grammes de PGGA, commercialisé par la Société Lubon sous le nom de "PGGA grade cosmétique", sont alors ajoutés lentement dans le vortex,

### Etape 2) : Ajustement du pH :

Le pH du mélange de l'étape 1) est ajusté à une valeur comprise entre 5,5 et 6,0 à l'aide d'une solution aqueuse d'HCl 5 M (température = 20°C),

### Etape 3) : Ajout du réticulant :

Dans le mélange de l'étape 2), sont additionnés 1,2 grammes de Polyéthylene glycol Diglycidyl Ether (PEGDGE) de poids moléculaire moyen de l'ordre de 500 g/mol,

### Etape 4) : Atomisation :

La solution aqueuse obtenue à l'étape 3) est concentrée par passage dans un atomiseur dont les paramètres opératoires sont les suivants :
- Débit d'introduction de la solution = 4 ml/min,
- Pression buse de sprayage = 1,5 bars,
- Température de l'air circulant = 150°C,

La composition (E1) est finalement isolée sous forme d'une poudre pulvérulente.

### Exemple 2 : Préparation d'un PGGA de sodium selon l'invention réticulé par du 1,4-Butanediol diglycidyl éther et atomisé :

Le procédé de synthèse de l'exemple 1 est reproduit en remplaçant les 1,2 grammes de Polyéthylene glycol Diglycidyl Ether (PEGDGE) par 0,48 gramme de 1,4-Butanediol Diglycidyl Ether commercialisé sous le nom d'ERISYS^{™} GE 21 par la Société EMERALD.

La composition (E2) obtenue est finalement isolée sous forme d'une poudre pulvérulente.

### Evaluation des PGGA de sodium réticulés et atomisés, obtenus dans les deux exemples précédents :

L'évaluation des compositions (E1) et (E2) (PGGA de sodium réticulés et atomisés), obtenues dans les deux exemples précédents, a été réalisée de la manière suivante :
- Dans un bécher de 400 ml forme haute, dispersion de 4 grammes des compositions à tester polymère sous forme de poudre pulvérulente dans 196 grammes d'eau sous agitation à l'aide d'un agitateur mécanique de marque Rayneri^{™} équipé d'un mobile de type défloculeuse jusqu'à obtention d'un gel homogène,
- Mesure de la viscosité dynamique à l'aide d'un viscosimètre de marque Brookfield et de type RVT Vitesse 5 en choisissant le mobile approprié,
- Ajout de 0,1% de NaCl sur le gel précédemment réalisé et agitation avec un agitateur mécanique de marque Rayneri^{™} équipé d'un mobile de type défloculeuse jusqu'à homogénéisation du mélange,
- Mesure de la viscosité dynamique à l'aide du même viscosimètre que précédemment toujours en choisissant le mobile approprié.

Un témoin a été réalisé à partir de PGGA non réticulé commercialisé par la Société Lubon sous le nom de "PGGA grade cosmétique".

Les valeurs de viscosité dynamique mesurées sont rassemblées dans le tableau ci-dessous :

**[Table 1]**

| Composition | Nature Réticulant | Viscosité en mPa.s du gel à 2% de polymère | Viscosité en mPa.s du gel à 2% de polymère + 0,1% de NaCl |
|---|---|---|---|
| Témoin^{a)} | Non | 176 (mobile 2) | 128 (mobile 2) |
| Composition (E1) | PEGDGE | 30 400 (mobile 5) | 9320 (mobile 3) |
| Composition (E2) | ERISYS^{™} GE 21 | 41 520 (mobile 5) | / |

| | | | |
|---|---|---|---|
| **^{a.}** PGGA non réticulé commercialisé par la Société Lubon sous le nom "PGGA grade cosmétique", Viscosités dynamiques de gels aqueux obtenus avec les compositions (E1) et (E2). | | | |

Les gels de PGGA de sodium témoin (non réticulé et non atomisé) présentent des viscosités comprises entre 100 et 200 mPa.s en présence ou non de NaCl.

En l'absence de NaCl, les gels de PGGA de sodium réticulés et atomisés possédent des viscosités respectivement égales à 30400 mPa.s dans le cas où le réticulant est le PEGDGE et à 41520 mPa.s avec l'ERISYS^{™} GE 21 comme réticulant.

En présence de NaCl, la viscosité du gel de PGGA de sodium réticulé par le PEGDGE et atomisé est égale à 9320 mPa.s.

Les compositions (E1) et (E2) selon l'invention permettent donc d'épaissir des phases aqueuses.

## Revendications

1. Procédé de préparation d'une composition pharmaceutique (F) à usage topique comprenant au moins un principe actif pharmaceutique et comme agent épaississant et pour 100% de sa masse, entre 0,1% et 10% massique d'un polymère (P) se présentant sous la forme d'un solide pulvérulent constitué d'unités monomériques issues d'acide glutamique (GA) partiellement ou totalement salifié, et d'unités monomériques d'au moins un agent réticulant (AR) portant au moins deux fonctions glycidyle, comprenant : une étape (A) de préparation d'un Polymère (P) comprenant les sous-étapes suivantes :
a) Une étape a) de préparation d'une phase polaire (PP) comprenant de l'acide polyglutamique, partiellement ou totalement salifié, au moins un solvant polaire (SP) choisi parmi les éléments du groupe constitué par l'eau, le méthanol, l'éthanol, le 1-propanol, le 2-propanol, l'iso butanol, le tertio-butanol, le 2-méthyl-2-propanol, le 1-butanol, le 2-butanol, l'acétone, le diméthyl cétone, le diéthyl cétone, le tétrahydropyrane, le tétrahydrofurane, le 2-méthyltétrahydrofurane, le 1,3-dioxane et le 1,4-dioxane et au moins un agent réticulant (AR) comprenant au moins deux fonctions glycidyles, choisi parmi les membres du groupe constitué par :
- le Monoéthylène Glycol Diglycidyl Ether de formule (I) : 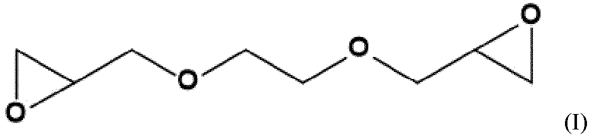
- le composé de formule (II) : 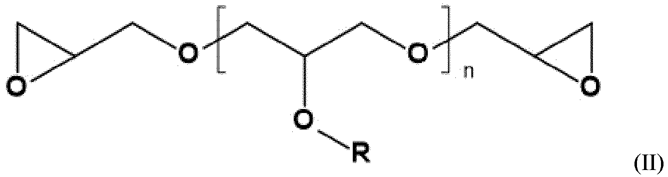 Avec R représentant un atome d'hydrogène ou le radical glycidyle 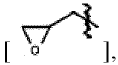 et n représentant un nombre entier supérieur ou égal à 1 et inférieur ou égal à 10 ;
- le 1,3-propanediol Diglycidyl Ether de formule (III) : 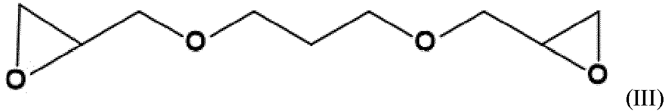
- Le 1,2-propanediol Diglycidyl Ether de formule (IV) : 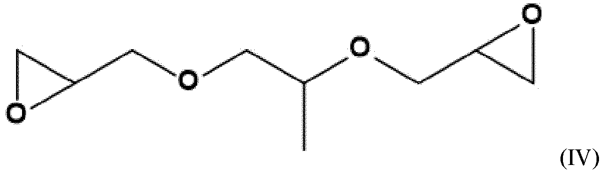
- le 1,4-butanediol Diglycidyl Ether de formule (V) : 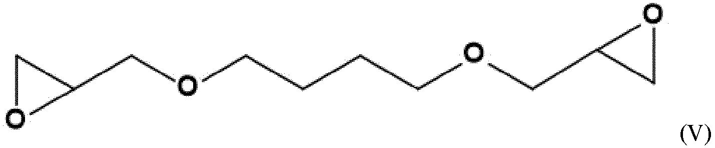
- le 1,2-butanediol Diglycidyl Ether de formule (VI) : 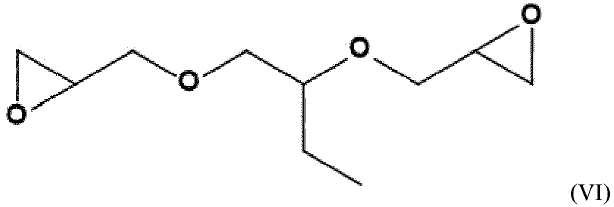
- Le 1,3-butanediol Diglycidyl Ether de formule (VII) : 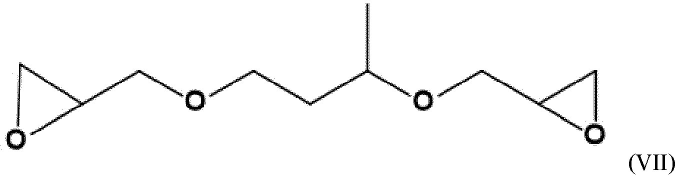
- Le 1,6-hexanediol Diglycidyl Ether de formule (VIII) : 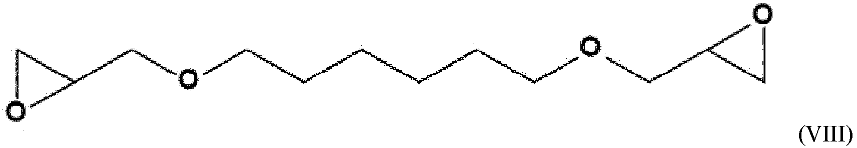
- Le composé de formule (IX) : 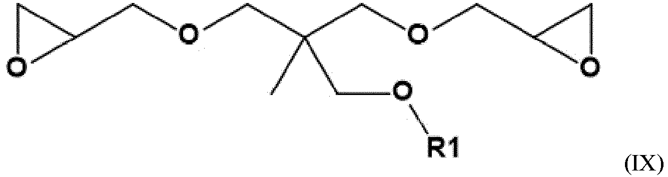 Avec R1 représentant un atome d'hydrogène ou le radical glycidyle 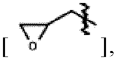
- Le composé de formule (X) : 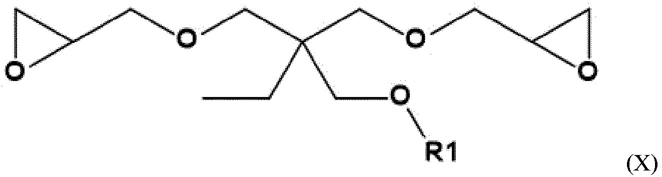 Avec R1 représentant un atome d'hydrogène ou le radical glycidyle 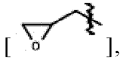
- Le composé de formule (XI) : 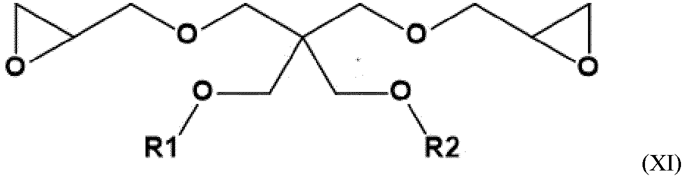 Avec R1 et R2, indépendants, représentant un atome d'hydrogène ou le radical glycidyle 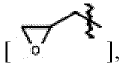
- Le composé de formule (XII) : 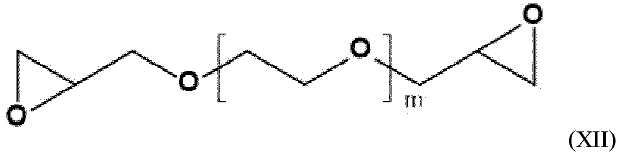 Avec m représentant un nombre entier supérieur ou égale à 2,
- Le composé de formule (XIII) : 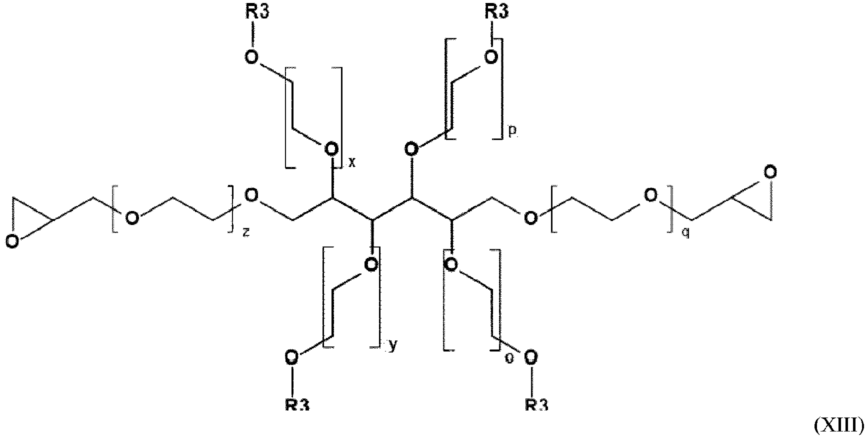 Avec R3 représentant un atome d'hydrogène ou le radical glycidyle 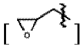 et x, y, z, o, p et q, indépendants les uns des autres, représentant un nombre entier supérieur ou égal à 2 et inférieur ou égal à 10 ;
b) Une étape b) d'ajustement du pH de la solution aqueuse obtenue à l'étape a) à un pH compris entre 3 et 11,
c) Une étape c) de séchage par atomisation de la phase polaire (PP) issue de l'étape b) au moyen d'un atomiseur mettant en œuvre un flux d'air et une buse dans lequel la température d'entrée du flux d'air est comprise entre 80°C et 180°C, de manière à obtenir le polymère (P) ;
Une étape (B) de mélange d'au moins un polymère (P) préparé lors de l'étape (A) avec au moins un principe actif pharmaceutique.

2. Procédé selon la revendication 1, **caractérisé en ce qu'**à l'étape a) l'acide polyglutamique est l'acide gamma-polyglutamique (PGGA).

3. Procédé selon la revendication 2, **caractérisé en ce qu'**à l'étape a) l'ensemble des unités monomériques constituant l'acide polyglutamique (PGA) est issu du glutamate de sodium, du glutamate de potassium, du glutamate d'ammonium, du glutamate de calcium, du glutamate de magnésium ou un mélange de ces formes.

4. Procédé selon l'une des revendications 1 à 3, **caractérisé en ce qu'**à l'étape a) la phase polaire (PP) comprend pour 100% de sa masse :
- De 5% à 80% massique d'acide polyglutamique,
- De 0,025% à 8% massique dudit au moins un agent de réticulant (AR),
- De 12% à 94,975% massique dudit au moins un solvant polaire (SP) .

5. Procédé selon l'une des revendications 1 à 4, **caractérisé en ce qu'**à l'étape a) le solvant polaire (SP) est l'eau.

6. Procédé selon la revendication 5, **caractérisé en ce qu'**à l'étape a) l'ensemble des unités monomériques constituant l'acide polyglutamique (PGA) est issu du glutamate de sodium et **en ce que** ledit agent réticulant (AR) est le Polyéthylèene glycol Diglycidyl Ether (PEDGE) ou le 1,4-Butanediol Diglycidyl Ether.

7. Procédé selon l'une des revendications 1 à 6, **caractérisé en ce qu'**à l'étape a) la phase polaire comprend en outre au moins un composé de formule (XIV): 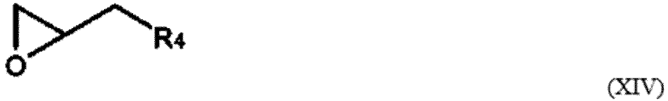 Avec R4 qui représente un radical hydrocarboné linéaire ou ramifié, saturé ou insaturée, comportant optionnellement au moins une fonction hydroxyle et comportant de six à 22 atomes de carbone choisi parmi les éléments du groupe constitué par le radical octyle, nonyle, décyle, undécyle, dodécyle, tridécyle, tétradécyle, pentadécyle, hexadécyle, heptadécyle, octadécyle, oléyle, linoléyle, linolényle, eicosyle, dodécosyle.

8. Procédé selon la revendication 7, **caractérisé en ce que** la teneur en composé de formule (XIV) dans la phase polaire (PP) est comprise, pour 100% massique de ladite phase polaire (PP), entre 0,05% et 35% massique, étant entendu que la somme des proportions massiques de l'acide polyglutamique, de l'agent de réticulation (AR), du solvant polaire (SP) et du composé de formule (XIV) est égale à 100%.

## Patentansprüche

1. Verfahren zur Herstellung einer topischen pharmazeutischen Zusammensetzung (F), umfassend mindestens einen pharmazeutischen Wirkstoff und als Verdickungsmittel und bezogen auf 100 % ihrer Masse, zwischen 0,1 % und 10 % Masseanteil eines Polymers (P), das in Form eines pulverförmigen Feststoffs vorliegt, der aus Monomereinheiten, die von teilweise oder vollständig salifizierter Glutaminsäure (GA) stammen, und Monomereinheiten von mindestens einem Vernetzungsmittel (AR), das mindestens zwei Glycidylfunktionen trägt, besteht, umfassend: einen Schritt (A) der Herstellung eines Polymers (P), der die folgenden Teilschritte umfasst:
a) Einen Schritt a) der Herstellung einer polaren Phase (PP), umfassend Polyglutaminsäure, teilweise oder vollständig salifiziert, mindestens ein polares Lösungsmittel (SP), ausgewählt aus den Elementen der Gruppe bestehend aus Wasser, Methanol, Ethanol, 1-Propanol, 2-Propanol, Isobutanol, tert-Butanol, 2-Methyl-2-propanol, 1-Butanol, 2-Butanol, Aceton, Dimethylketon, Diethylketon, Tetrahydropyran, Tetrahydrofuran, 2-Methyltetrahydrofuran, 1,3-Dioxan und 1,4-Dioxan, und mindestens ein Vernetzungsmittel (AR), das mindestens zwei Glycidylfunktionen umfasst, ausgewählt aus den Mitgliedern der Gruppe bestehend aus:
- Monoethylenglycoldiglycidylether der Formel (I): 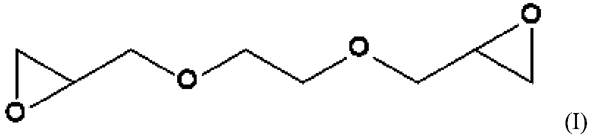
- der Verbindung der Formel (II): 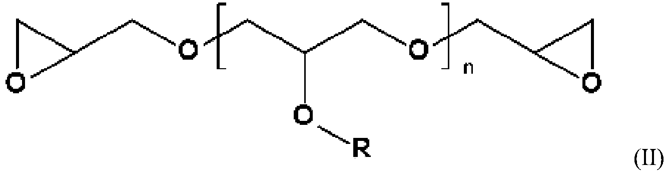 Wobei R ein Wasserstoffatom oder den Glycidylrest 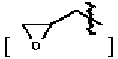 darstellt und n eine ganze Zahl größer oder gleich 1 und kleiner oder gleich 10 darstellt;
- 1,3-Propandioldiglycidylether der Formel (III): 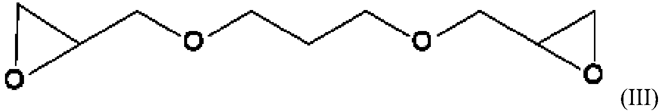
- Dem 1,2-Propandioldiglycidylether der Formel (IV): 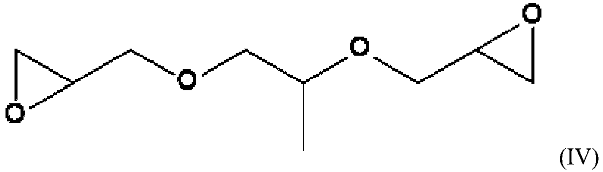
- dem 1,4-Butandioldiglycidylether der Formel (V): 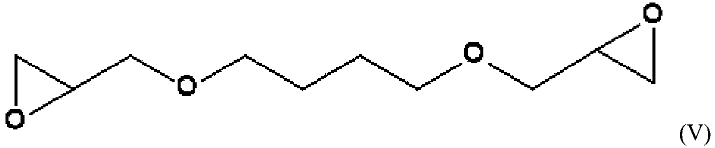
- dem 1,2-Butandioldiglycidylether der Formel (VI): 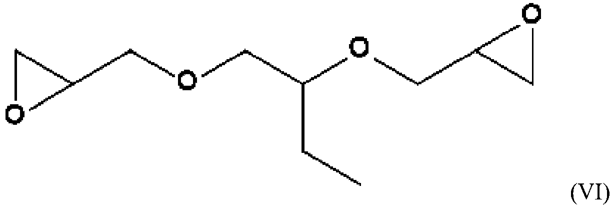
- Dem 1,3-Butandioldiglycidylether der Formel (VII): 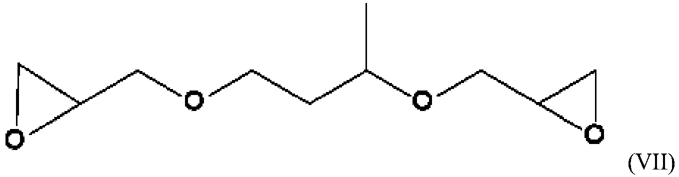
- Dem 1,6-Hexandioldiglycidylether der Formel (VIII): 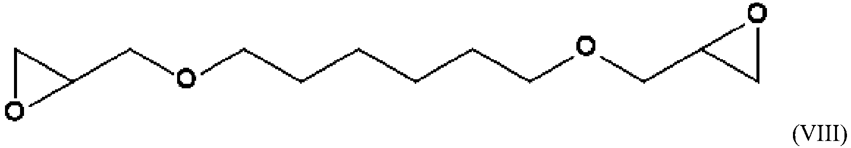
- Der Verbindung der Formel (IX): 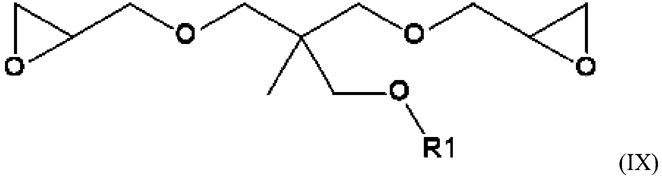 Wobei R1 ein Wasserstoffatom oder den Glycidylrest 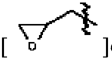 darstellt,
- Der Verbindung der Formel (X): 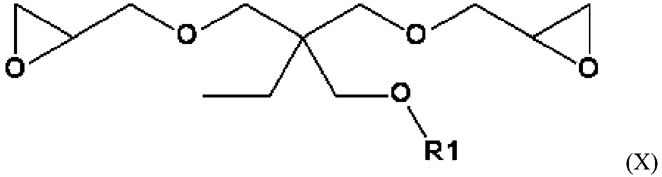 Wobei R1 ein Wasserstoffatom oder den Glycidylrest 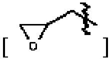 darstellt,
- Der Verbindung der Formel (XI): 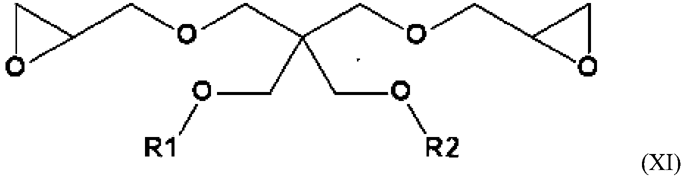 Wobei R1 und R2, unabhängig voneinander, ein Wasserstoffatom oder den Glycidylrest [ 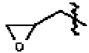 ] darstellen,
- Der Verbindung der Formel (XII): 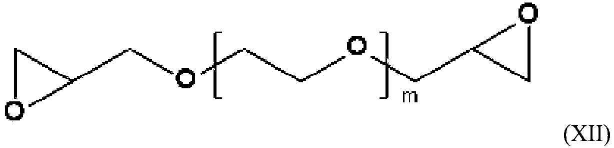 Wobei m eine ganze Zahl größer oder gleich 2 darstellt,
- Der Verbindung der Formel (XIII): 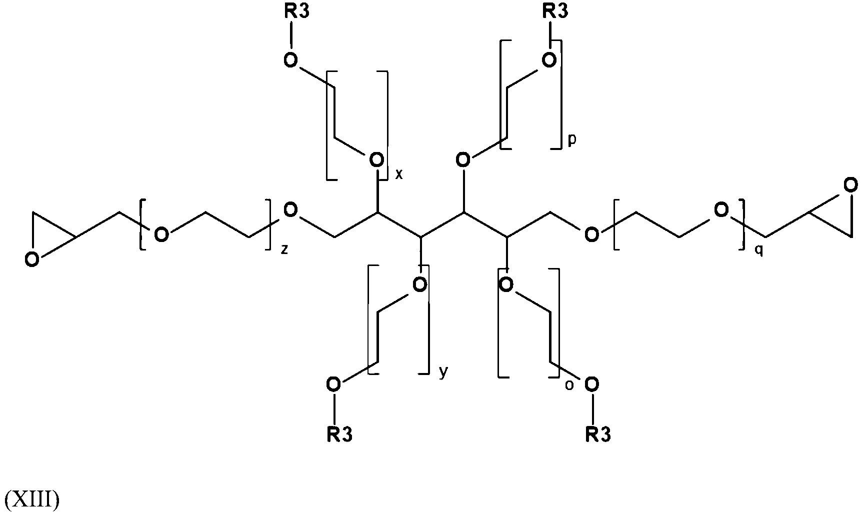
Wobei R3 ein Wasserstoffatom oder den Glycidylrest darstellt und x, y, z, o, p und q, unabhängig voneinander, eine ganze Zahl größer oder gleich 2 und kleiner oder gleich 10 darstellen;
b) Einen Schritt b) der Einstellung des pH-Werts der in Schritt a) erhaltenen wässrigen Lösung auf einen pH-Wert zwischen 3 und 11,
c) Einen Schritt c) der Trocknung durch Zerstäubung der aus Schritt b) stammenden polaren Phase (PP) mittels eines Zerstäubers, der einen Luftstrom und eine Düse einsetzt, wobei die Eintrittstemperatur des Luftstroms zwischen 80°C und 180°C liegt, um das Polymer (P) zu erhalten;
Einen Schritt (B) des Mischens mindestens eines während des Schritts (A) hergestellten Polymers (P) mit mindestens einem pharmazeutischen Wirkstoff.

2. Verfahren nach Anspruch 1, **dadurch gekennzeichnet, dass** in Schritt a) die Polyglutaminsäure Gamma-Polyglutaminsäure (PGGA) ist.

3. Verfahren nach Anspruch 2, **dadurch gekennzeichnet, dass** in Schritt a) die Gesamtheit der Monomereinheiten, die die Polyglutaminsäure (PGA) bilden, aus Natriumglutamat, Kaliumglutamat, Ammoniumglutamat, Calciumglutamat, Magnesiumglutamat oder einer Mischung dieser Formen stammt.

4. Verfahren nach einem der Ansprüche 1 bis 3, **dadurch gekennzeichnet, dass** in Schritt a) die polare Phase (PP) für 100 % ihrer Masse umfasst:
- Von 5 % bis 80 % Masseanteil an Polyglutaminsäure,
- Von 0,025 % bis 8 % Masseanteil des mindestens einen Vernetzungsmittels (AR),
- Von 12 % bis 94,975 % Masseanteil des mindestens einen polaren Lösungsmittels (SP).

5. Verfahren nach einem der Ansprüche 1 bis 4, **dadurch gekennzeichnet, dass** in Schritt a) das polare Lösungsmittel (SP) Wasser ist.

6. Verfahren nach Anspruch 5, **dadurch gekennzeichnet, dass** in Schritt a) die Gesamtheit der Monomereinheiten, die die Polyglutaminsäure (PGA) bilden, aus Natriumglutamat stammt und dass das genannte Vernetzungsmittel (AR) Polyethylenglycoldiglycidylether (PEDGE) oder 1,4-Butandioldiglycidylether ist.

7. Verfahren nach einem der Ansprüche 1 bis 6, **dadurch gekennzeichnet, dass** in Schritt a) die polare Phase zusätzlich mindestens eine Verbindung der Formel (XIV) umfasst: 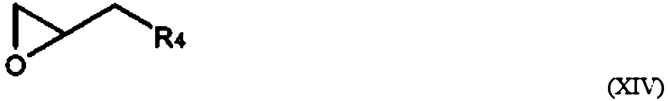 Wobei R4, das einen linearen oder verzweigten, gesättigten oder ungesättigten Kohlenwasserstoffrest darstellt, der optional mindestens eine Hydroxylfunktion aufweist und sechs bis 22 Kohlenstoffatome aufweist, ausgewählt aus den Elementen der Gruppe bestehend aus dem Octyl-, Nonyl-, Decyl-, Undecyl-, Dodecyl-, Tridecyl-, Tetradecyl-, Pentadecyl-, Hexadecyl-, Heptadecyl-, Octadecyl-, Oleyl-, Linoleyl-, Linolenyl-, Eicosyl-, Dodecosylrest.

8. Verfahren nach Anspruch 7, **dadurch gekennzeichnet, dass** der Gehalt an der Verbindung der Formel (XIV) in der polaren Phase (PP), für 100 % Masseanteil der polaren Phase (PP), zwischen 0,05 % und 35 % Masseanteil liegt, mit der Maßgabe, dass die Summe der Masseanteile der Polyglutaminsäure, des Vernetzungsmittels (AR), des polaren Lösungsmittels (SP) und der Verbindung der Formel (XIV) gleich 100 % ist.

## Claims

1. A method for preparing a topical pharmaceutical composition (F) comprising at least one active pharmaceutical ingredient and as a thickening agent and for 100% of its mass, between 0.1% and 10% by mass of a polymer (P) in the form of a pulverulent solid consisting of monomeric units derived from glutamic acid (GA) that is partially or totally salified, and monomeric units of at least one cross-linking agent (AR) bearing at least two glycidyl functions, comprising: a step (A) of preparing a Polymer (P) comprising the following substeps:
a) A step a) of preparing a polar phase (PP) comprising polyglutamic acid, partially or totally salified, at least one polar solvent (SP) chosen from the elements of the group consisting of water, methanol, ethanol, 1-propanol, 2-propanol, isobutanol, tert-butanol, 2-methyl-2-propanol, 1-butanol, 2-butanol, acetone, dimethyl ketone, diethyl ketone, tetrahydropyran, tetrahydrofuran, 2-methyltetrahydrofuran, 1,3-dioxane and 1,4-dioxane and at least one cross-linking agent (AR) comprising at least two glycidyl functions, chosen from the members of the group consisting of:
- Monoethylene Glycol Diglycidyl Ether of formula (I): 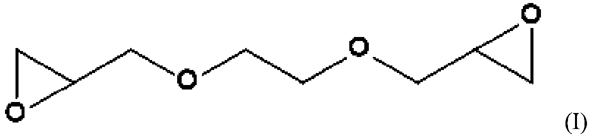
- the compound of formula (II): 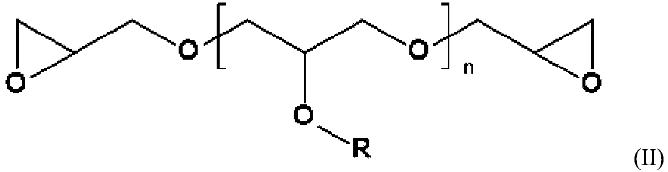 With R representing a hydrogen atom or the glycidyl radical 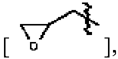 and n representing an integer greater than or equal to 1 and less than or equal to 10;
- 1,3-propanediol Diglycidyl Ether of formula (III): 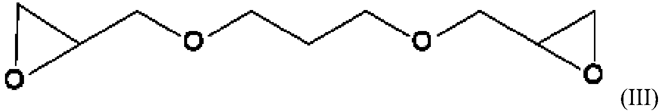
- The 1,2-propanediol Diglycidyl Ether of formula (IV): 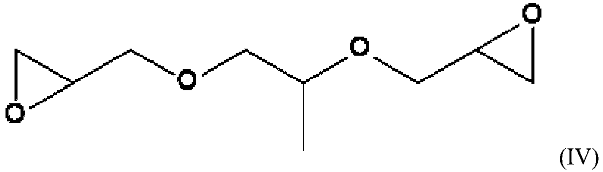
- the 1,4-butanediol Diglycidyl Ether of formula (V): 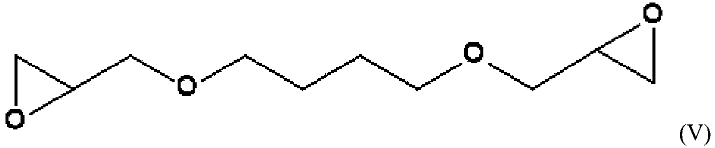
- the 1,2-butanediol Diglycidyl Ether of formula (VI): 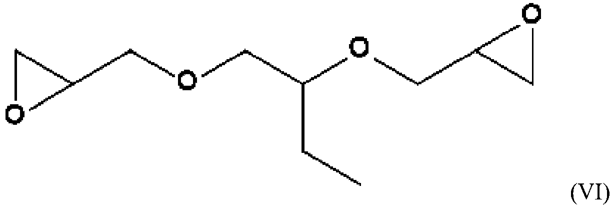
- The 1,3-butanediol Diglycidyl Ether of formula (VII): 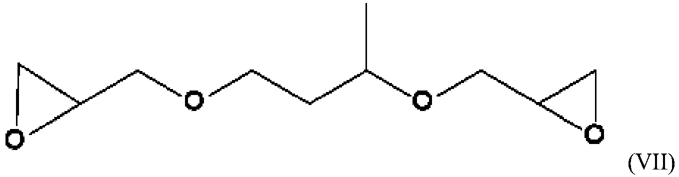
- The 1,6-hexanediol Diglycidyl Ether of formula (VIII): 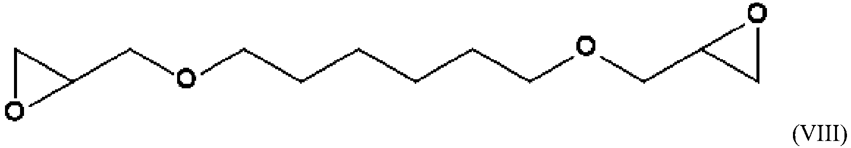
- The compound of formula (IX): 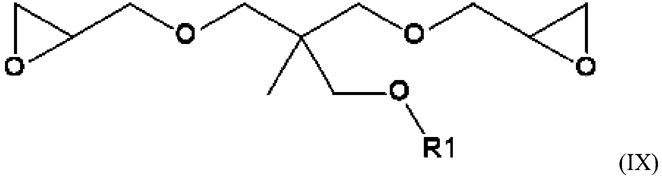 With R1 representing a hydrogen atom or the glycidyl radical 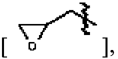
- The compound of formula (X): 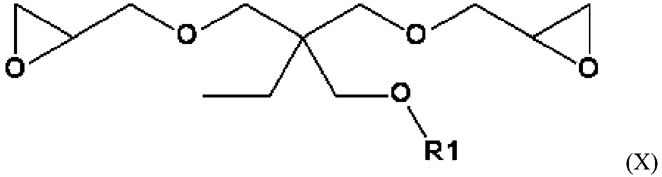 With R1 representing a hydrogen atom or the glycidyl radical 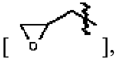
- The compound of formula (XI): 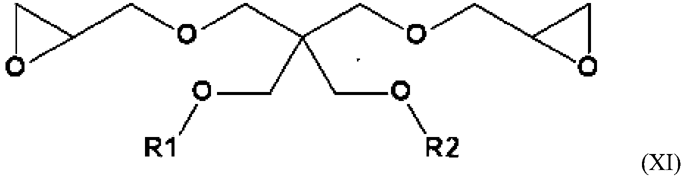 With R1 and R2, independently, representing a hydrogen atom or the glycidyl radical [ 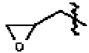 ],
- The compound of formula (XII): 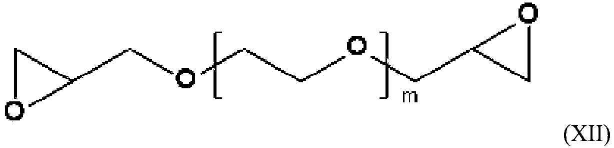 With m representing an integer greater than or equal to 2,
- The compound of formula (XIII): 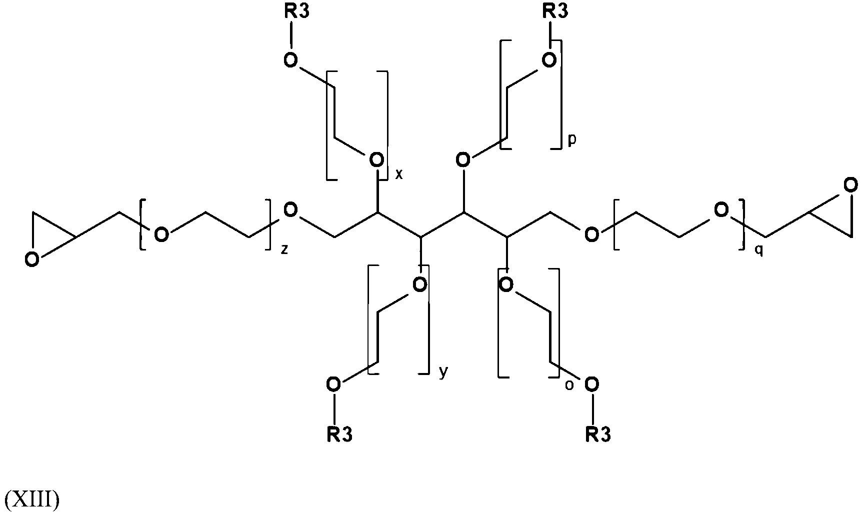
With R3 representing a hydrogen atom or the glycidyl radical and x, y, z, o, p and q, independently of one another, representing an integer greater than or equal to 2 and less than or equal to 10;
b) A step b) of adjusting the pH of the aqueous solution obtained in step a) to a pH between 3 and 11,
c) A step c) of drying by atomization of the polar phase (PP) resulting from step b) by means of an atomizer implementing an air flow and a nozzle in which the inlet temperature of the air flow is between 80°C and 180°C, so as to obtain the polymer (P);
A step (B) of mixing at least one polymer (P) prepared during step (A) with at least one active pharmaceutical ingredient.

2. The method according to claim 1, **characterized in that** in step a) the polyglutamic acid is gamma-polyglutamic acid (PGGA).

3. The method according to claim 2, **characterized in that** in step a) all the monomeric units constituting the polyglutamic acid (PGA) are derived from sodium glutamate, potassium glutamate, ammonium glutamate, calcium glutamate, magnesium glutamate or a mixture of these forms.

4. The method according to one of claims 1 to 3, **characterized in that** in step a) the polar phase (PP) comprises for 100% of its mass:
- From 5% to 80% by mass of polyglutamic acid,
- From 0.025% to 8% by mass of said at least one cross-linking agent (AR),
- From 12% to 94.975% by mass of said at least one polar solvent (SP).

5. The method according to one of claims 1 to 4, **characterized in that** in step a) the polar solvent (SP) is water.

6. The method according to claim 5, **characterized in that** in step a) all the monomeric units constituting the polyglutamic acid (PGA) are derived from sodium glutamate and **in that** said cross-linking agent (AR) is Polyethylene Glycol Diglycidyl Ether (PEDGE) or 1,4-Butanediol Diglycidyl Ether.

7. The method according to one of claims 1 to 6, **characterized in that** in step a) the polar phase further comprises at least one compound of formula (XIV): 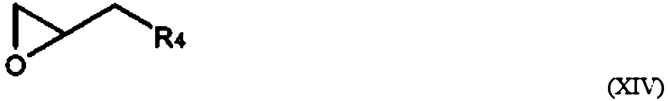 With R4 which represents a linear or branched, saturated or unsaturated hydrocarbon radical, optionally comprising at least one hydroxyl function and comprising from six to 22 carbon atoms chosen from the elements of the group consisting of the octyl, nonyl, decyl, undecyl, dodecyl, tridecyl, tetradecyl, pentadecyl, hexadecyl, heptadecyl, octadecyl, oleyl, linoleyl, linolenyl, eicosyl, dodecosyl radicals.

8. The method according to claim 7, **characterized in that** the content of the compound of formula (XIV) in the polar phase (PP) is comprised, for 100% by mass of said polar phase (PP), between 0.05% and 35% by mass, it being understood that the sum of the mass proportions of the polyglutamic acid, of the cross-linking agent (AR), of the polar solvent (SP) and of the compound of formula (XIV) is equal to 100%.
